# EUROPEAN PATENT APPLICATION

(11) **EP 1 142 907 A1**
(43) Date of publication of application: **10.10.2001**
(21) Application number: 99959429.4
(22) Date of filing: 22.12.1999
(51) Int. Cl.: C07K 14/435, C12N 15/12

(54) **THE POLYPEPTIDE DREAM, USED AS Ca2+ IONS-DEPENDENT TRANSCRIPTIONAL REGULATOR AND ITS GENE CODING THEREFOR**

(30) Priority: 22.12.1998 ES 9802667; 22.12.1999 ES 9902825
(71) Applicant: CONSEJO SUPERIOR DE INVESTIGACIONES CIENTIFICAS, 28006 Madrid (ES)
(72) Inventor: NARANJO OROVIO, José Ramon, Inst. Neurobiologia, Calle Doctor Arce, 37, E-28002 Madrid (ES); MELLSTROM, Britt, Inst. Neurobiologia, Calle Doctor Arce, 37, E-28002 Madrid (ES); DOMPABLO GARCIA, Isidro, Insto. Neurobiologia, Calle Doctor Arce, 37, E-28002 Madrid (ES); LINK, Wolfgang, Alexander, Insto. Neurobiologia, Calle Doctor Arce, 37, E-28002 Madrid (ES); CARRION RODRIGUEZ, Angel Manuel, Inst. Neurobiolog, Calle Doctor Arce, 37, E-28002 Madrid (ES); LEDO GOMEZ, Francisco, Insto. Neurobiologia, Calle Doctor Arce, 37, E-28002 Madrid (ES)
(74) Representative: Elzaburu Marquez, Alberto
(86) International application number: ES9900402
(87) International publication number: WO0037498

(57) **Abstract**

The repression of DREAM of DRE-dependent gene expression (c-jun and c-fos between DREAM (EFmutDREAM and LVmutDREAM) escape from blocking by Ca²⁺ and by others) is regulated by Ca²⁺ and by the protein αCREM. Mutated αCREM forms, respectively, and make it possible to define new strategies for therapeutic control of pathologic processes of the oncologic type, learning disorders, etc. On the other hand, DREAM is a target protein for the identification of new therapeutic compounds. Moreover, the mutated forms in the EF-hand regions of DREAM (EFmutDREAM) block the activity of the CRE-dependent proteins, and can be used in the regulation of the expression of these genes, CREB and CREM, involved in the final activation of numerous genes.

## Description

### 2. Industrial sector

Biomedicine: Control of the expression of genes with DRE and CRE sequences.

### 3. State of the art

The flows of intracellular Ca²⁺ are important determinants of gene expression (Bading, H., Ginty, D.D. & Greenberg, M.E. Regulation of gene expression in hippocampal neurons by distinct calcium signaling pathways. *Science* **260,** 181-186 (1993); Ghosh, A. & Greenberg, M.E. Calcium signaling in neurons: molecular mechanisms and cellular consequences. *Science* **268,** 239-247 (1995); Hardingham, G.E., Chawla, S., Johnson, C.M. & Bading, H. Distinct functions of nuclear and cytoplasmic calcium in the control of gene expression. *Nature* **385,** 260-265 (1997); Dolmetsch, R.E., Xu, K. & Lewis, R.S. Calcium oscillations increase .the efficiency and specificity of gene expression. *Nature* **392,** 933-936 (1998); Li. W. et al. Cell-permeant caged InsP₃ ester shows that Ca²⁺ spike frequency can optimize gene expression. *Nature* **392,** 936-941 (1998)). Up to now, it has been considered that calcium-regulated phosphatases and kinases are involved in changes through phosphorylation of transcription factors characterized by their binding to specific DNA sequences. The binding of these transcription factors to these regulatory elements would modulate the expression of certain target genes (Sheng, M., Thompson, M.A. & Greenberg, M.E. CREB: a Ca²⁺-regulated transcription factor phosphorylated by calmodulindependent kinases. *Science* **252,** 1427-1430 (1991); Dolmetsch, R.E., Lewis, R.S., Goodnow, C.C. & Healy, J.I. Differential activation of transcription factors induced by Ca²⁺-response amplitude and duration. *Nature* **386,** 855-858 (1997); Johnson, C.H., Hill, C.S., Clawla, S., Treisman, R. & Bading, H. Calcium controls gene expression via three distinct pathways that can function independently of the Ras/Mitogen activated protein kinase (ERKs) signaling cascade. *J. Neurosci.* **17,** 6189-6202 (1997)). On the other hand, the existence of agents that are direct effectors of Ca²⁺-regulated gene expression has been postulated (Ghosh, A. & Greenberg, M.E. Calcium signalling in neurons: molecular mechanisms and cellular consequences. *Science* **268,** 239-247 (1995)) but none of them has been identified so far.

The regulatory element DRE (downstream regulatory element) is a transcription silencer that is independent of orientation, first described in the promoter of the human prodynorphin gene (Carrión, A.M., Mellström, B. & Maranjo, J.R. PKA-dependent derepression of the human prodynorphin gene via differential binding to an intragenic silencer element. Mol. Cell. Biol. 18:6921-6929 (1998)). Binding to the DRE site causes blocking of prodynorphin expression and directed mutagenesis of the DRE site leads to the appearance of high levels of transcripts of this gene. DNA-protein binding experiments (southwestern) suggest the existence of a protein complex that binds specifically to the DRE. However, no polypeptide with these characteristics has been identified so far.

### 4. Description of the invention

### 4.1. Brief description

Repression of DREAM of DRE-dependent gene expression (c-jun and c-fos between DREAM (EFmutDREAM and LVmutDREAM) escape blocking by Ca²⁺ and by others) is regulated by Ca²⁺ and by the protein αCREM. Mutated forms αCREM, respectively, and make it possible to define new strategies for therapeutic control of pathologic processes of the oncologic type, learning disorders, etc. On the other hand, DREAM is a target protein for the identification of new therapeutic compounds. Moreover, the mutated forms in the EF-hands regions of DREAM (EFmutDREAM) block the activity of the CRE-dependent proteins, and can be used in regulation of the expression of these CREB and CREM genes, which are involved in the final activation of numerous genes. The invention also relates to:
the recombinant vectors that contain this gene and code for the DREAM polypeptide with transcription repressor activity that can be modulated,
the recombinant vectors that contain dominant negative mutants of this gene and code for the mutated DREAM polypeptide with transcription repressor activity that cannot be modulated,
the transformed cells with this gene, in which it is possible to identify blocking agents or activators of the DREAM polypeptide and evaluate the activity of the said agents in DRE-dependent gene expression,
the transformed cells with negative dominant mutants of this gene in which it is possible to block DRE-dependent gene expression permanently and regulate CRE-dependent transcription,
the polyclonal or monoclonal antibodies directed against the said DREAM polypeptide, which can be used for analytical purposes for purifying the said polypeptide, for *in vitro* diagnosis, or for therapeutic purposes, especially for disorders involving cell differentiation, apoptosis or autoimmunity, and
the recombinant vectors of specific cellular expression that contain this gene and code for the DREAM polypeptide or its negative dominant mutants with transcription repressor activity that can be modulated or is permanent, respectively, and their use in gene therapy for producing selective gene silencing of cell populations that are liable to proliferation as tumours, to degeneration, to autoimmune processes or to processes of hyperstimulation triggering an altered physiologic response (epilepsy, learning disorders, etc.).

### 4.2. Detailed description

Expression of the human prodynorphin gene is regulated by derepression at the level of the DRE regulatory sequence that acts as a silencer element (Carrión, A.M., Mellström, B. & Naranjo, J.R. PKAdependent derepression of the human prodynorphin gene via differential binding to an intragenic silencer element. Mol. Cell. Biol. 18:6921-6929 (1998)). To isolate proteins that bind to the DRE element we used a double-chain oligonucleotide (SEQ ID NO1) containing a tandem of two consecutive DRE sequences. Once labelled, this oligonucleotide was used as "primer" for functional probing of a gene library of human caudate by standard methods.

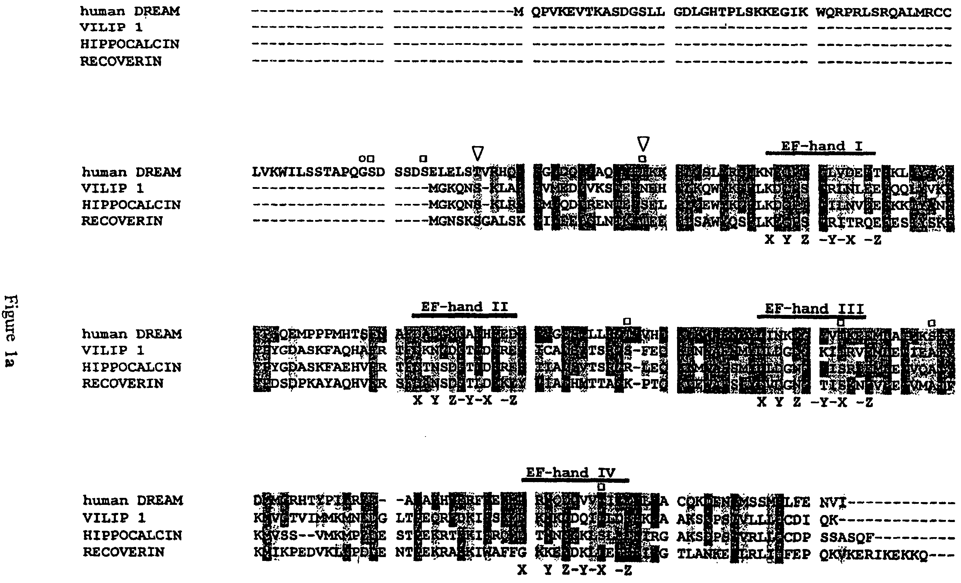

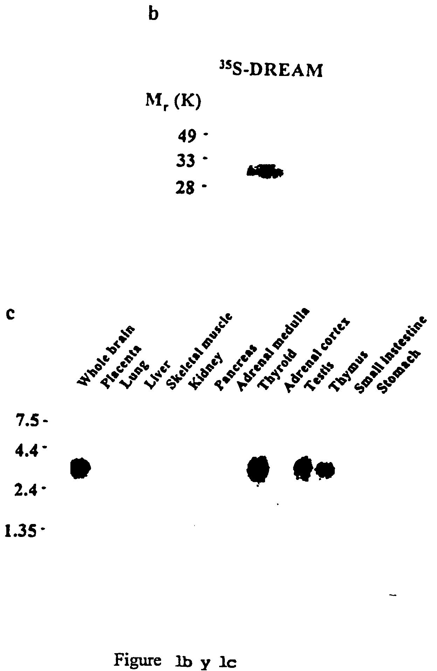

Thus, we obtained a complementary DNA (SEQ ID NO2) that encodes a human protein of 256 amino acids (SEQ ID NO3) which we call DREAM (DRE Antagonist Modulator) with a theoretical molecular weight of 31.8 kDa. By means of *in vitro* translation of the cDNA in a rabbit reticulocyte system, we confirmed that a protein of the expected size was obtained (Fig. 1a). In RNA analysis experiments we observed that the cDNA of DREAM hybridized with a messenger of approximately 3.0 kb, widely represented in the brain, thymus, thyroid and testes (Fig. 1b).

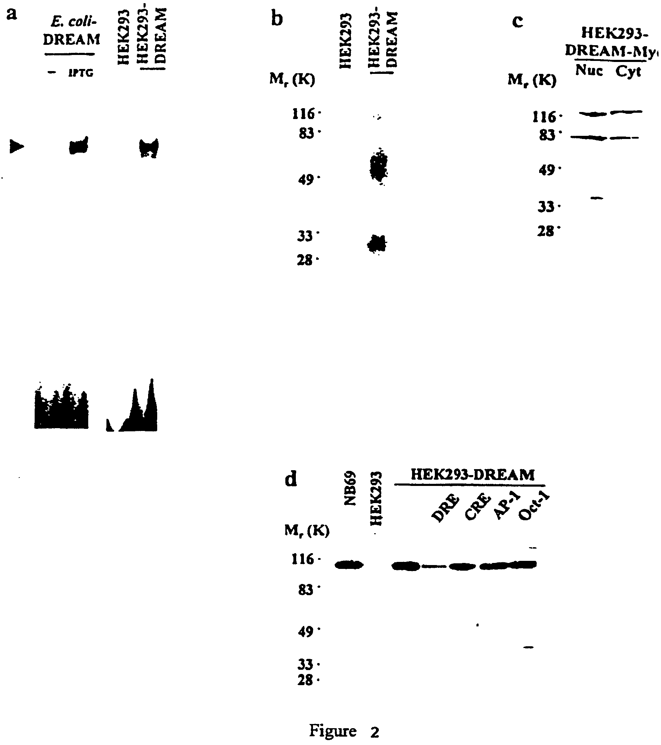

Next we set out to confirm that this new DREAM protein binds to DRE. Overexpression of the cDNA of DREAM was effected in bacteria and in HEK293 eukaryotic cells; both these systems lack endogenous expression of DREAM. The ability to bind to the DRE sequence of the extracts of this DREAM protein was analysed by various tests of protein-DNA interaction: delay gel, crosslinking tests and south-western blotting (Fig. 2). Thus, it was demonstrated that this protein binds to the DRE sequence (Fig. 2a), that DREAM interacts with the DNA either as monomer, dimer or tetramer (Fig. 2c), and that the affinity for DRE is greater in the tetrameric configuration (Fig. 2d). Moreover, it is interesting to note that binding of the DREAM tetramer to the DRE sequence does not compete with oligonucleotides containing CRE, AP-1 or OCT1 sequences (Fig. 2d). As the DRE sequence functions as a silencer, we have called this DRE-binding protein "DREAM" (DRE Antagonist Modulator).

Furthermore, starting from human DREAM protein purified from NB69 neuroblastoma cells or recombinant DREAM protein expressed in bacteria, we proceeded to the development and production of polyclonal antibodies in the rabbit and of mouse hybridomas that produce DREAM-specific monoclonal antibodies. This polyclonal antibody was used in subsequent western blots. The presence of monomers, dimers or tetramers was confirmed by western blots using this antibody against the DREAM protein or an antibody against a Myc epitope capable of recognizing the DREAM-Myc fusion protein (Fig. 2c). Using this technique, it was also observed that DREAM is distributed both in the nucleus and in the cytoplasm (Fig. 2c). It should be pointed out that these antibodies can be used for analytical purposes for purifying the said polypeptide, for *in vitro* diagnosis or for blocking this polypeptide for therapeutic purposes.

Analysis of the amino acid sequence of this protein revealed the presence of four domains of binding to calcium known as EF-hands (Nakayama, S. & Kretsinger, R.H. Evolution of the EF-hand family of proteins. *Annu. Rev. Biophys. Biomol. Struct.* **23,** 473-507 (1994)) (SEQ ID NO4, SEQ ID NO5, SEQ ID NO6, and SEQ ID NO7).

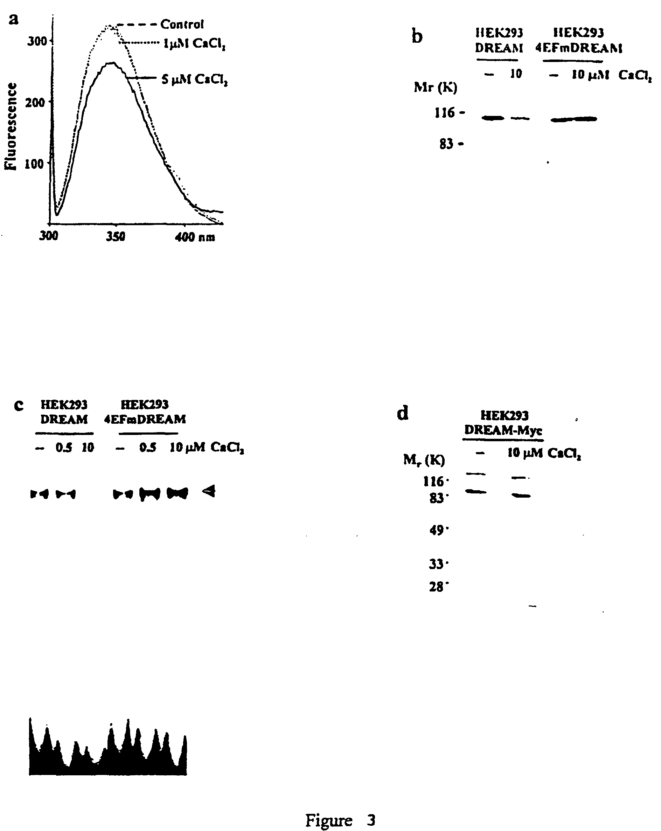

To investigate the functional significance of these EF-hand domains in DREAM we analysed the tryptophan fluorescence emission spectrum of this protein in the presence of and absence of calcium. Addition of 5 µM Ca²⁺ decreases the fluorescence signal (Fig. 3A), indicating that DREAM binds specifically to Ca²⁺. Furthermore, using the technique of directed mutagenesis we prepared a series of mutated DREAMs: single, double or triple (2, 3, 4, 2/3, 2/4, 3/4 and 2/3/4EFmutDREAM) in which the first (X) and third (Y) residues responsible for binding to Ca²⁺ in the second, third and fourth EF-hands domain were replaced by alanine (SEQ ID NO8, SEQ ID NO9 and SEQ ID NO10). Analysis of DREAM and 4EFmutDREAM by southwestern and delay gel showed some similar results (Fig. 3B and 3C). However, on adding 10 µM of Ca²⁺ we observed that in the nuclear extracts containing unmutated DREAM there was a significant drop in intensity of the 110K band, whereas this addition of Ca²⁺ did not have any kind of effect in the case of the mutated form of DREAM (4EFmutDREAM). Similar results were observed with other mutated forms of EF-DREAM (data not presented). These results suggest that the binding of Ca²⁺ to the EF-hand region induces a conformational change of DREAM that prevents its binding to the DRE sequence without affecting the stability of the tetramer of DREAM.

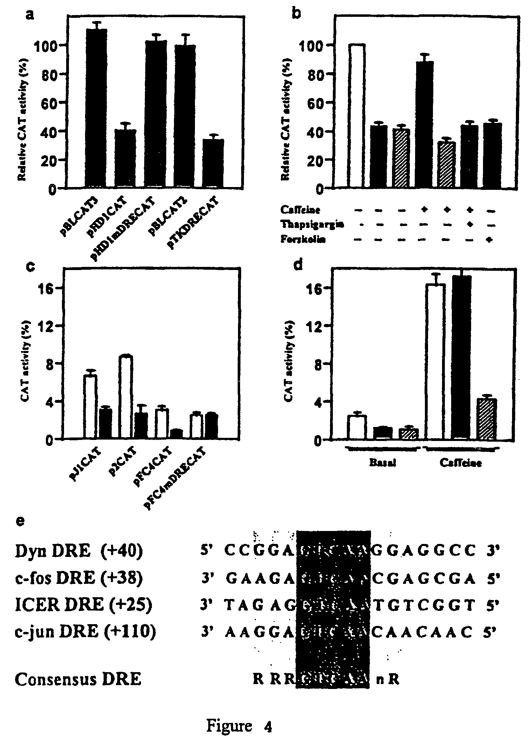

It has been described that the DRE sequence functions as a silencer in the promoter of the human prodynorphin gene (Carrión, A.M., Mellström, B. & Naranjo, J.R. PKA-dependent derepression of the human prodynorphin gene via differential binding to an intragenic silencer element. *Mol. Cell. Blol*. **18:**6921-6929 (1998)). To evaluate the potential regulatory activity of DREAM transcription via the DRE sequence, a transient transfection test was carried out with the promoter of the human prodynorphin gene (pHD1CAT) or with constructions of heterologous promoters (pTKDRECAT) and a DREAM expression vector. Cotransfection with DREAM significantly represses the basal transcription of both promoters in HEK293 cells, whereas the basal transcription of the empty reporters pBLCAT3 and pBLCAT2 is not altered (Fig. 4a). Moreover, DREAM failed to repress the transcription of the reporter plasmid pHD1mDRECAT, in which the DRE endogenous site had been mutated (Fig. 4a). These results suggest that the DREAM protein functions as a repressor transcription factor through binding to the DRE sequence. In fact, blocking of expression of the endogenous DREAM in cells of NB69 human neuroblastoma by the induction of stable expression of the complementary chain of the messenger RNA of DREAM (SEQ ID 6) assumes a drop in levels of DREAM protein and a significant increase in the expression of genes regulated negatively by DREAM, concretely the prodynorphin gene in these cells (data not presented). This opens up the possibility of using transient gene suppression of DREAM as a way of increasing, temporally and selectively, the expression of target genes of DREAM in various tissues.

Next we evaluated the effect of Ca²⁺ in transcription repression mediated by DREAM by comparing the transcription response of natural DREAM and its mutated form (EFmutDREAM) after the release of intracellular Ca²⁺ induced by caffeine (Hernández-Cruz, A., Sala, F. & Adams, P.R. Subcellular calcium transients visualized by confocal microscopy in a voltage-clamped vertebrate neuron. *Science* **147,** 858-862 (1990)). Cotransfection with DREAM and 4EFmutDREAM gives similar results in repression of the function of the reporter in basal conditions (Fig. 4b), consistent with the earlier finding that 4EFmutDREAM is still able to bind to the DRE sequence (Fig. 3b and 3c). However, whereas increase in intracellular Ca²⁺ induced by caffeine completely suppresses the repressor effect of DREAM on transcription (Fig. 4b), caffeine does not alter the repression exerted by the mutated form of DREAM (4EFmutDREAM) as a consequence of its inability to respond to an increase in Ca²⁺. The same results were obtained with other single, double and triple mutated forms of DREAM (EFmutDREAMs) (data not presented), indicating that any mutations in these EF-hand regions are sufficient to suppress the effect of stimulation by Ca²⁺. This derepression effect of caffeine is directly related to release of Ca²⁺ on verifying that it is blocked by thapsigargin and rianodin, drugs that block the release of intracellular calcium (Ehrlich, B.E., Kaftan, E., Bezprozvannaya, S. and Bezprozvannaya, I. The pharmacology of intracellular Ca²⁺-release channels. *TIPS* **15,** 145-149 (1994)) (Fig. 4b). Moreover, this derepression by caffeine is not related to the inhibitory effect of phosphodiesterases and the subsequent increase in intrinsic cAMP on treatment with caffeine since the direct activation of PKA by forskolin does not affect the repression of pTKDRECAT by DREAM in HEK293 cells (Fig. 4b).

The next step was to check whether the DREAM protein represses the transcription of other genes activated by Ca²⁺. For this we focused on three early induction genes previously reported as regulated by Ca²⁺: *c-fos*, *c-jun* and *ICER* (Morgan, J.I. & Curran, T. Role of ion flux in the control of *c-fos* expression. *Nature* **322,** 552-555 (1986); Naranjo, J.R. et al. Peripheral noxious stimulation induces CREM expression in dorsal horn: Involvement of glutamate. *Eur. J. Neurosci.* **9,** 2778-2783 (1997)). The first evidence of this was perhaps obtained on analysing the more 3' zone of the promoters of these three genes, when it was found that in all cases there was a DRE element immediately 3' to the initiation site of transcription. To evaluate whether these DRE sites were functional, we carried out transient cotransfection of HEK293 cells with DREAM and an FC4CAT reporter (which contained the c-fos promoter, (Sassone-Corsi, P. Sisson, J.C. & Verma, I.M. Transcriptional autoregulation of the proto-oncogene *fos. Nature* **334,** 314-319 (1988). )) or a J1CAT reporter (which contained the promoter of c-jun, deGroot, R.P., Pals, C. & Kruijer, W. Transcriptional control of c-jun by retinoic acid. *Nucleic Acid Res*. **19,** 1585-1591 (1991)) or a p2CAT reporter (which contained the P2 promoter of the CREM gene, Molina, C., Foulkes, N.S., Lalli, E. & Sassone-Corsi, P. Inducibility and negative autoregulation of CREM: an alternative promoter directs the expression of ICER, an early response repressor. *Cell* **75,** 875-886 (1993) (Fig. 4c). Thus, it was found that the basal expression of these three reporters was reduced with DREAM (Fig. 4c) whereas in the case of the pFC4mutDRECAT reporter containing a mutation at the DRE site of the promoter of c-fos, its basal expression was not repressed by DREAM (Fig. 4c). These results indicate that DREAM causes basal repression of these three early induction genes, acting via the DRE sequences. It should be added that activation of the c-fos reporter by the increase in intracellular Ca²⁺ after administration of caffeine was blocked on using a DREAM negative dominant mutant (4EFmutDREAM) whereas repressor action of DREAM was not observed in the presence of an increase in intracellular Ca²⁺ (Fig. 4d).

To summarize, the three c-fos, c-jun and ICER early genes regulated by DREAM act as "master" genes, regulating in their turn the expression of numerous target genes. In addition, we should emphasize the important role of these three genes in the control of cell proliferation, differentiation and/or death, from which it is inferred that regulation by DREAM and/or its mutated forms, of the expression of these genes implicates DREAM in the control of such important aspects as cell proliferation, differentiation and death in tumour-forming, degenerative and autoimmune processes and in learning disorders. This offers the possibility of using DREAM and/or its negative dominant mutants in gene therapy of these pathologic processes.

Other central mediators of the nuclear response to cAMP and to Ca²⁺ are the bZip activating or repressing transcription factors that have the ability to bind to the cAMP-regulated promoter element (CREs, cAMP responsive promoter elements) (Hoeffler, J.P., Meyer, T.E., Yun, Y., Jameson, J.L. and Habener, J.F. Cyclic AMP-responsive DNA-binding protein: structure based on a cloned placental cDNA. *Science* **242,** 1430-1433 (1988); Foulkes, N.S., Borrelli, E. & Sassone-Corsi, P. CREM gene: use of alternative DNA-binding domains generates multiple antagonists of cAMP-induced transcription. *Cell* ***64,*** 739-749 (1991); Molina, C., Foulkes, N.S., Lalli, E. & Sassone-Corsi, P. Inducibility and negative autoregulation of CREM: an alternative promoter directs the expression of ICER, an early responsive repressor. *Cell* **75**, 875-886 (1993)). This family of transcription factors includes proteins coded by the CREB and CREM genes, the activator function of which is closely regulated by phosphorylation by means of a wide range of various kinases that includes PKA and CaMKinases (Laoide, B.M., Foulkes, N.S., Schlotter, F. & Sassone-Corsi, P. The functional versatility of CREM is determined by its modular structure. *EMBO J.* **12,** 1179-1191 (1993); Gonzalez, G.A., Yamamoto, K.K., Fischer, W.H., Karr, D., Menzel, P., Biggs III, W.H., Vale, W.W. & Montminy, M.R. A cluster of phosphorylation sites on cyclic AMP-regulated nuclear factor CREB predicted by its sequence. *Nature* **337**, 749-752 (1989); Groot, R.P., Hertog, J., Vandenheede, J.R., Goris, J. & Sassone-Corsi, P. Multiple and cooperative phosphorylation events regulate the CREM activator function. *EMBO J.* **12,** 3903-3911 (1993)). All in all, these proteins represent the convergence of various signal cascades. Moreover, after derepression of the DRE sites observed after treatment with forsfokolin we are interested in determining whether the CREM or CREB proteins can interact functionally with DREAM and analysing its possible role in this derepression.

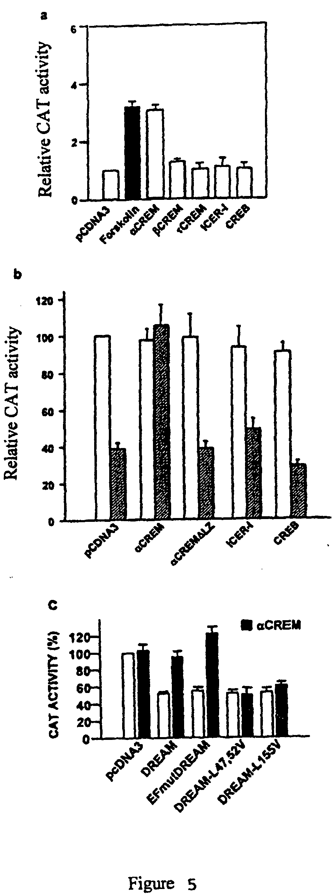

To determine the role of the possible interactions between the DREAM protein and the CREB and CREM proteins we undertook cotransfection experiments with these proteins. Thus, we conducted experiments of cotransfection in NB69 cells with the plasmid pHD3CAT, which contains binding sites for DRE but not CRE (Carrión, A.M., Mellström, B. & Naranjo, J.R. PKA-dependent derepression of the human prodynorphin gene via differential binding to an intragenic silencer element. Moll. Cell. Biol. **18,** 6921-6929 (1998)), together with expression vectors for CREB or for the various isomorphs of CREM. We found that the overexpression of CREM simulated the effect of forsfokolin and that it induced the expression of the pHD3CAT reporter (Fig. 5a). This effect was specific for αCREM as none of the other isomorphs of CREM or CREB was able to repress pHD3CAT (Fig. 5a). To exclude the direct effect of binding of CREM to the pHD3CAT reporter, similar experiments were carried out in HEK293 cells, a cell line that does not express DREAM (Carrión, A.M., Link, W.A., Ledo, F., Mellström, B. & Naranjo, J.R. DREAM is a Ca²⁺-regulated transcriptional repressor. *Nature* **398,** 80-84 (1999)). Overexpression of DREAM in HEK293 cells represses the basal expression of the pHD3CAT reporter, as was described previously (Carrión, A.M., Link, W.A., Ledo, F., Mellström, B. & Naranjo, J.R. DREAM is a Ca²⁺-regulated transcriptional repressor. *Nature* **398,** 80-84 (1999)), whereas overexpression of αCREM alone failed to alter the basal levels of expression of pHD3CAT (Fig. 5b). However, when they were cotransfected jointly αCREM repressed completely the repression by DREAM (Fig. 5b). It should be emphasized that deletion of the terminal region of α CREM which includes the bZip domain for binding and dimerization to DNA (αCREMΔLZ) did not block DREAM-mediated repression (Fig. 5b). On the other hand, we did not observe derepression of the pHD3CAT reporter after cotransfection with isoforms of ICER (I, Iγ , II or IIγ) (Fig. 5b and other data not presented), which are generated starting from the alternative promoter P2 with the terminal 3' portion of the CREM gene. The ICER proteins incorporate not only the bZip domain of CREM but moreover do not have the N terminal region of αCREM (Foulkes, N.S., Borrelli, E., & Sassone-Corsi, P. CREM gene: use of alternative DNA-binding domains generate multiple antagonists of cAMP-induced transcription. Cell, **64**, 739-749 (1991); Molina, C., Foulkes, N.S., Lalli, E. & Sassone-Corsi, P. Inducibility and negative autoregulation of CREM: an alternative promoter directs the expression of ICER, an early responsive repressor. *Cell* **75,** 875-886 (1993); Laoide, B.M., Foulkes, N.S., Schlotter, F. & Sassone-Corsi, P. The functional versatility of CREM is determined by its modular structure. *EMBO J.* **12,** 1179-1191 (1993)). Interestingly, CREB also failed to derepress pHD3CAT even after activation of PKA after forsfocolin (Fig. 5b and data not presented). These data are in agreement with the data observed in the NB69 cells and indicate that both N and C-terminal regions of αCREM are involved in functional interaction with DREAM that displaces that of the DRE sites.

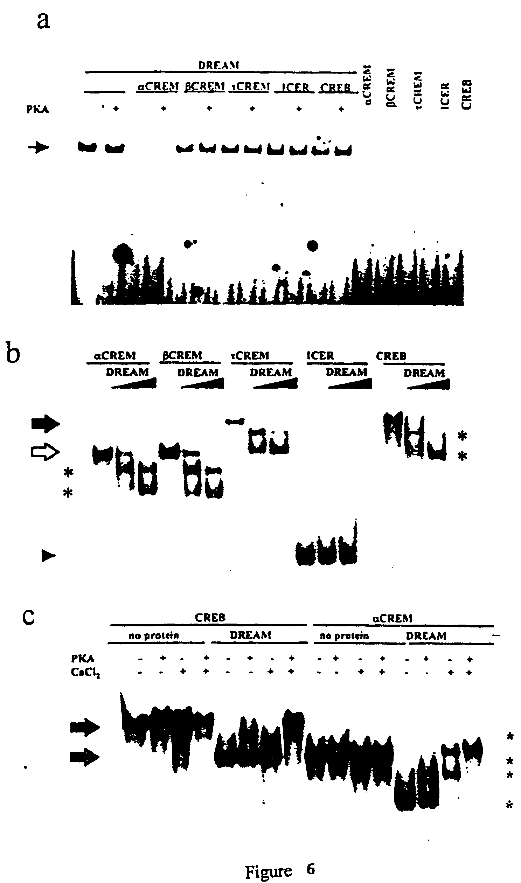

To evaluate the specific interaction of DREAM-α CREM, these recombinant proteins expressed in bacteria were analysed by retardation experiments using DRE as probe. The results permit us to conclude that αCREM does not bind to the DRE probe, but the formation of DREAM-αCREM heteromers displaces DREAM from the DRE sites, completely eliminating the DREAM-αCREM delay band (Fig. 6a). In agreement with the results of cotransfection (Fig. 5), the ICER-I recombinant protein, which is equivalent to truncation of the N-terminal region of αCREM, did not block the DREM-DRE delay band (Fig. 6a). Moreover, the C-terminal region of αCREM is essential for this interaction, as in other isomorphs of CREM, such as βCREM which differs from α CREM in the alternative use of a second bZip domain, did not block the DREAM-DRE delay band either (Fig. 6a). Thus, both N and C terminal regions of αCREM participate in the interaction with DREAM in blocking its binding to DRE.

Since phosphorylation of the serine 68 residue at the N-terminal end of αCREM is an important determinant of its activity (Laoide, B.M., Foulkes, N.S., Schlotter, F. & Sassone-Corsi, P. The functional versatility of CREM is determined by its modular structure. *EMBO J.* **12,** 1179-1191 (1993); Groot, R.P., Hertog, J., Vandenheede, J.R., Goris, J. & Sassone-Corsi, P. Multiple and cooperative phosphorylation events regulate the CREM activator function. *EMBO J.* **12,** 3903-3911 (1993), we decided to analyse whether this affected interaction with DREAM. The *in vitro* phosphorylation of αCREM by protein kinase A (Nichols, M., Weih, F., Schmid, W., DeVack, C., Kowenz-Leutz, E., Luckow, B., Boshart, M. & Schültz, G. Phosphorylation of CREB affects its binding to high and low affinity sites: implications for cAMP-induced gene transcription. *EMBO J.,* **11,** 3337-3346 (1992)) increased the efficiency in blocking of the DREAM-DRE delay band (Fig. 6a). The effect of PKA was blocked by coincubation with H89, a selective inhibitor of PKA (data not presented). In contrast, CREB and the other isomorphs of CREM were incapable of blocking the DREAM-DRE delay band, even after its phosphorylation (Fig. 6a). In accordance with the absence of sites of phosphorylation by PKA in DREAM (Carrión, A.M., Link, W.A., Ledo, F., Mellström, B. & Naranjo, J.R. DREAM is a Ca²⁺-regulated transcriptional repressor. *Nature* **398,** 80-84 (1999)) the incubation of DREAM with PKA did not alter binding to DRE (Fig. 6a). These results all imply that the specific protein-protein interaction between DREAM and αCREM causes loss of the ability of DREAM to bind to DRE.

In addition, we verified that DREAM-αCREM binding does not interfere in the binding of αCREM to the CRE sites. For this, we undertook some similar binding experiments using CRE as probe. We did not observe CRE delay bands after the incubation of increasing amounts of DREAM recombinant protein with the CRE probe (Fig. 6b). However, DREAM greatly altered the migration of the αCREM delay band without resulting in blocking it (Fig. 6b). Interestingly, migration of the CRE delay bands formed with other isomorphs of CREM or CREB changed in the presence of DREAM. However, DREAM did not alter the CRE delay bands obtained with recombinant ICER (Fig. 6b and data not presented). These results suggest a second type of CRE-binding protein complexes with DREAM for which only the N-terminal activator region of the CREB or CREM proteins is necessary. In these cases, the complexes still maintain their ability to bind to the DRE and CRE sites, indicating that this interaction does not directly affect the domain for binding to DNA. This effect of DREAM in the CRE delay bands was dependent on the amount of DREAM (Fig. 6b and 6c) with a partial delay observed with smaller amounts of DREAM (Fig. 6b). These results suggest that various multimer complexes form between DREAM and CRE-binding proteins, and are capable of binding to CRE.

Next we analysed whether phosphorylation of the CREB or CREM proteins alters their ability to interact with DREAM via their N-terminal. In-vitro phosphorylation of the CREB or αCREM recombinant protein by PKA only partially represses the delay induced by DREAM, indicating a decrease in interaction between the two (Fig. 6c). This effect of phosphorylation was also observed when PKA was added to the DREAM-CREM or DREAM-CREB complexes already formed, indicating that the protein-protein interaction does not prevent the phosphorylation of CREB or CREM by PKA (data not observed). Previously it was observed that binding of Ca²⁺ to the EF-hands of DREAM alters its conformation, blocking its ability to bind to DRE sites. We therefore analysed whether the presence of Ca²⁺ has an effect on the formation of the protein complexes between DREAM and CREB. Addition of Ca²⁺ to the incubation reaction did not alter the CRE delay bands derived from CREM or CREB but reduced the capacity of DREAM to delay the said bands (Fig. 6c). Interestingly, the combined effect of Ca²⁺ and of phosphorylation by PKA completely reversed the displacement induced by DREAM of the DRE delay bands (Fig. 6c), suggesting that in the presence of Ca²⁺, DREAM does not interact with the phosphorylated CREM or CREB proteins.

To analyse whether interaction between DREAM and the CREM or CREB proteins occurs *in vivo,* we carried out a series of experiments of coimmunoprecipitation of the native proteins starting from nuclear extracts of rat brain. In accordance with the results of cotransfection and delay gels CREB was immunoprecipitated from these rat brain extracts using a rat anti-DREAM antibody. On the other hand, competition of the rat anti-DREAM antibody with recombinant DREAM protein completely blocked its ability to immunoprecipitate CREB. Moreover, immunoprecipitation of CREB with the anti-DREAM antibody was not observed in cellular extracts of liver, a tissue that does not exhibit endogenous expression of DREAM (Carrión, A.M., Link, W.A., Ledo, F., Mellström, B. & Naranjo, J.R. DREAM is a Ca²⁺- regulated transcriptional repressor. *Nature* **398,** 80-84 (1999)).

As already described, the strategy of blocking the derepression of genes involved in cell proliferation processes (tumour-forming processes, inflammatory processes, etc.) and regulated by DRE by the use of mutated forms of DREAM (EFmutDREAM) that does not respond to intracellular Ca²⁺ is of therapeutic interest. Of course, following this therapeutic line and with the result described in the present invention that the αCREM form is able to block the gene repression capacity of DREAM, it is interesting to identify the region or regions of DREAM involved in protein-protein interaction with αCREM for their subsequent genetic manipulation by mutation enables it to escape from interaction with αCREM. Studies of directed mutagenesis that were carried out enabled us to establish the involvement of certain amino acids of the N-terminal region of DREAM and of the region between the second and third EF-hands in interaction with αCREM. For definitive establishment of the possible role of these regions, cotransfection experiments were carried out in HEK293 cells with mutated forms of DREAM in these regions (DREAM L47-52V, mutated form of DREAM in these amino acids of the N-terminal region and DREAM L155V, a form mutated in the region between the 2nd and 3rd EF-hands, where the amino acids leucine 47, 52 and 155 were replaced by valine, which are called LVmutDREAM hereinafter (SEQ ID N12 and SEQ ID N13, respectively) (Fig. 5c). Thus, it was found that the mutated form of DREAM (EFmutDREAM), which prevents derepression by Ca²⁺, is displaced from its binding to DRE by αCREM (Fig. 5c). However, the mutated forms described in the present invention (DREAM L47-52V and DREM L155V) do not interact with αCREM and therefore its capacity for gene repression via DRE is unaffected (Fig. 5c). It should be pointed out that these new mutated forms of DREAM (LVmutDREAM) are sensitive to derepression by Ca²⁺ (data not presented).

These new mutated forms of DREAM (LVmutDREAM) are concrete examples of the group of possible mutated forms of DREAM that do not interact with αCREM and that remain unaffected in their capacity for gene repression of the genes previously mentioned and they all form a real option for gene therapy of those pathologies whose aetiopathogenesis is due to the expression of these genes and form part of the present invention. Of course, mutated forms of DREAM that combine the mutations described in the EF-hands with those described in the present invention will not be affected in their repressor function by any of the stimuli mentioned: Ca²⁺ and intracellular cAMP, and are more powerful genetic tools than isolated mutated forms and form part of the present invention.

On the other hand, as was mentioned previously, the identification of compounds that simulate the effect of these mutations that have been described (LVmutDREAM) and prevent protein-protein interaction between DREAM and αCREM is of interest for its therapeutic potential in the pathologies described above. The transformed cells that are described in the present invention can provide a basis for a method of identification of these compounds from ability to compare the activity of DREAM before and after administration of the said compounds. This method of identification of new therapeutic compounds, antitumour and anti-inflammatory among others, forms a part of the present invention.

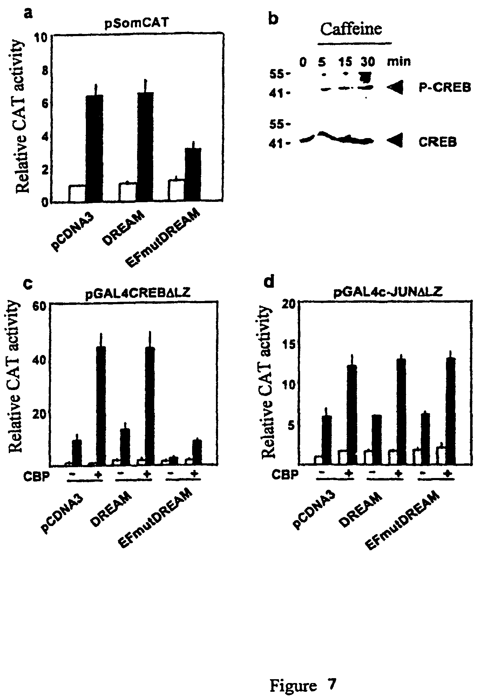

Next we analysed the consequences of interaction between DREAM and CREB in the expression of CRE-dependent genes. For this we conducted cotransfection experiments using pSOMCAT, a reporter plasmid that includes a canonical CRE site within the promoter of the somatostatin gene (Sassone-Corsi, P., Visvader, J., Ferland, L., Mellon, P.L. & Verma, I.M. Induction of protooncogene c-fos transcription through the adenylate cyclase pathway: characterization of cAMP responsive element. Genes Dev. 2, 1529-1538 (1988)). The increase in Ca²⁺ levels after treatment with caffeine (Carrión, A.M., Link, W.A., Ledo, F., Mellström, B. & Naranjo, J.R. DREAM is a Ca²⁺-regulated transcriptional repressor. *Nature* **398,** 80-84 (1999); Hernández-Cruz, A., Sala, F. & Adams, P.R. Subcellular calcium transients visualized by confocal microscopy in a voltage-clamped vertebrate neuron. *Science* **147,** 858-862 (1990)) caused a six-fold increase in transactivation of this reporter plasmid after cotransfection in HEK293 cells (Fig. 7a). Cotransfection with the native forms of DREAM (wtDREAM) did not affect the basal activity of the pSOMCAT plasmid and did not change after treatment with caffeine (Fig. 7a). However, cotransfection with mutated forms of DREAM (EFmutDREAM), a negative dominant mutant that does not respond to Ca²⁺, significantly reduced the transactivation of the CRE reporter after administration of caffeine (Fig. 7a). A sharp and sustained increase in phosphorylated CREB protein occurs in the HEK293 cells after caffeine (Fig. 7b) and is probably responsible for the transactivation of the pSOMCAT reporter. These results further reinforce the *in vitro* data indicating that both changes together, phosphorylation of CREB and modification of DREAM after Ca²⁺, are necessary for complete rupture of the DREAM-CREB complex so that complete induction of the CRE promoter is obtained. In control tests using pTRETKCAT, a reporter plasmid that contains an AP-1 site of the promoter of the collagenase gene (Sassone-Corsi, P., Lamp, W.W., Kamps, M. & Verma, I.M. Fos-associated cellular p39 is related to nuclear transcription factor AP-1. *Cell* **54,** 553-560 (1988)), neither the native DREAM (wtDREAM) nor the mutated forms (EFmutDREAM) alter the transactivation of the AP-1 reporter induced by caffeine (data not presented) indicating that repression by DREAM mediated by Ca²⁺ is CRE-specific.

The transactivation of the CRE reporters after phosphorylation of CREB depends on their ability to recruit the transcriptional coactivator CBP (CREB-binding protein) (Chrivia, J.C., Kwok, R.P.S., Lamb, N., Hagiwara, M., Montminy, M.R. & Goodman, R.H. Phosphorylated CREB binds specifically to the nuclear protein CBP. *Nature* **365,** 855-859 (1993); Kwok, R.P.S., Lundblad, J.R., Chrivia, J.C., Richards, J.P., Bächinger, H.P. Brennan, R.G., Roberts, S.G.E., Green, M.R. & Goodman, R.H. Nuclear protein CBP is a coactivator for the transcription factor CREB. *Nature* **370**, 223-226 (1994)). Thus, we wondered whether the decrease in transactivation of the CREB-EFmutDREAM complexes of the CRE sites would be associated with less capacity of the phosphorylated form of CREB to recruit CBP when it is forming complexes with DREAM. To investigate this possibility we carried out cotransfection with the pG5CAT reporter plasmid, that contains five GAL4 binding sites, with the GAL4-CREBΔLZ fusion protein and the transcriptional coactivator CBP in HEK293 cells. We compared the effect of release of intracellular Ca²⁺ after caffeine (Carrión, A.M., Link, W.A., Ledo, F., Mellström, B. & Naranjo, J.R. DREAM is a Ca²⁺-regulated transcriptional repressor. *Nature* **398,** 80-84 (1999); Hernández-Cruz, A., Sala, F. & Adams, P.R. Subcellular calcium transients visualized by confocal microscopy in a voltage-clamped vertebrate neuron. *Science* **147**, 858-862 (1990)) in the presence of the native form (wtDREAM) or negative dominant mutated form of DREAM (EFmutDREAM). Use of the GAL4-CREBΔLZ fusion protein, which exhibits absence of the bZip domain for DNA-binding/dimerization (ΔLZ), together with the pG5CAT reporter, eliminates the possibility of interference from endogenous CREB protein. Cotransfection of pGAL4-CREBΔLZ with pCBP caused an up to 45-fold increase in the basal levels of transactivation after treatment with caffeine as a result of phosphorylation of CREB (Sheng, M., Thompson, M.A. & Greenberg, M.E. CREB: a Ca²⁺-regulated transcription factor phosphorylated by calmodulin-dependent kinases. *Science* **252,** 1427-1430 (1991); Impey, S., Obrietan, K., Wong, S.T., Poser, S., Yano, S., Wayman, G., Deloulme, J.C., Chan, G. & Storm, D.R. Cross talk between ERK and PKA is required for Ca²⁺ stimulation of CREB-dependent transcription and ERK nuclear translocation. *Neuron* **21,** 869-883 (1998); Yamamoto, K.K., Gonzalez, G.A., Biggs III, W.H., & Montminy, M.R. Phosphorylation-induced binding and transcriptional efficacy of nuclear factor CREB. *Nature* **334,** 494-498 (1988)) and CBP (Chawla, S., Hardingham, G.E., Quinn, D.R. & Bading, H. CBP: a signal regulated transcriptional coactivator controlled by nuclear calcium and CaM kinase IV. *Science* **281,** 1505-1509 (1998); Hardingham, G.E., Chawla, S., Cruzalegui, F.H. and Bading, H. Control of recruitment and transcription-activating function of CBP determines gene regulation by NMDA receptors and L-type calcium channels. *Neuron 22,* 789-798 (1999); Hu, S-C., Chrivia, J. & Ghosh, A. Regulation of CBP-mediated transcription by neuronal calcium signalling. *Neuron* **22,** 799-808 (1999)) dependent on calcium and the subsequent interaction of these two phosphoproteins for inducing the transactivation of the reporter pGSCAT (Fig. 7c). A similar capacity for transactivation was observed after caffeine treatment in the presence of the native form of DREAM (wtDREAM) (Fig. 7c). However, in cultures transfected with the mutant form of DREAM (EFmutDREAM), transactivation of the pG5CAT reporter by GAL4-CREBΔ LZ/CBP decreased dramatically (Fig. 7c). Activation, by caffeine, of the endogenous expression of CBP or its homologue p300, in HEK293 cells, showed similar repression with the mutated forms of DREAM (EFmutDREAM) though with lower levels (Fig. 7c). Control tests using the vector pGAL4 did not show increased levels of transactivation of the pG5CAT reporter after treatment with caffeine and/or cotransfection with the expression vectors (data not presented). All in all, these results indicate that interaction between DREAM and the CREB proteins is calcium-dependent and can block the capacity of the phosphorylated CREB protein to recruit the activated CBP protein.

To elucidate the possibility of the existence of interaction between DREAM and CBP, some similar tests were carried out, using the GAL4-cJunΔLZ fusion protein. Treatment with caffeine increased the transactivation of the pG5CXAT reporter by the GAL4-cJunΔLZ protein between 5 and 12 times the basal levels either in the presence or absence of endogenous CBP, respectively (Fig. 7d). In these tests, however, cotransfection of the native or mutated form of DREAM did not produce any effect (Fig. 7d). Since CREB and c-Jun interact with the same domain of CBP (Kwok, R.P.S., Lundblad, J.R., Chrivia, J.C., Richards, J.P., Bächinger, H.P., Brennan, R.G., Roberts, S.G.E., Green, M.R. & Goodman, R.H. Nuclear protein CBP is a coactivator for the transcription factor CREB. *Nature* **370,** 223-226 (1994); Arias, J., Alberts, A.S., Brindle, P., Claret, F.X., Smeal, T., Karin, M., Feramisco, J. & Montminy, M.R. Activation of cAMP and mitogen responsive genes relies on a common nuclear factor. *Nature* **370,** 226-229 (1994)) these results suggest that there is no direct interaction between DREAM and CBP and that Ca²⁺-dependent repression of the capacity for induction of CRE-mediated transcription by CBP is due exclusively to the ability of DREAM to interact with CREB.

These results indicate that in basal conditions DREAM modulates the activity of promoters that contain CRE sites of a Ca²⁺-dependent form by forming protein-protein complexes with CREB and isomorphs of CREM. On the other hand, cAMP-mediated derepression of DRE is specifically mediated by interaction between DREAM and αCREM. It can therefore be postulated that the interaction of DREAM with transcription factors that bind to CRE sites is a point of interaction between the cascades of Ca²⁺- and cAMP-dependent intracellular signals in the regulation of gene expression. Thus, the mutated forms of DREAM (EFmutDREAM) that interact with CREB and CREM and do not respond to Ca²⁺ block the activity of these proteins, i.e. they alter the regulation of gene expression that these transcription factors exert on numerous CRE-dependent genes and form part of the present invention.

### Detailed description of the drawings

Fig. 1. The DREAM protein and its messenger RNA. a, Amino acid sequence of the human DREAM protein indicating the positions of the EF domains. Comparison with other proteins containing EF domains. b, In vitro expression of the ³⁵S-labelled DREAM protein using the system of lysates of rabbit reticulocytes. c, Northern blot analysis of the distribution of the transcripts of DREAM in various human tissues. The markers of molecular weight for RNA (in kb) are shown on the left of the diagram.

Fig. 2. DREAM binds to DRE. a, Test of the delay-gel type where the specific delayed DRE band is observed (arrow) after overexpression of DREAM in *E. coli* and in HEK293 cells. b, UV crosslinking test of the DRE probe and nuclear extracts of cells with overexpression of DREAM (HEK293-DREAM). Three bands corresponding to the monomer, dimer and tetramer of DREAM are observed. c, Western blot test of nuclear (Nuc) and cytoplasmic (Cyt) extracts of HEK293 cells with overexpression of the DREAM-Myc fusion protein. d, Southwestern test with nuclear extracts of HEK293-DREAM cells, it can be seen that DREAM binds to DRE as a 110K complex. The 110K band obtained with endogenous DREAM from nuclear extracts of NB69 cells is shown for comparison. In addition, the competition of unlabelled oligonucleotides containing DRE, CRE, AP-1 or Oct-1 sequences is shown.

Fig. 3. DREAM is a protein that binds to calcium. a, Fluorescence emission spectrum of 0.5 µM DREAM 1 in absence of calcium (dashed line) and in presence of 1 µ M (dotted line) or 5 µM (solid line) of calcium. b, Southwestern test after stable overexpression of DREAM or 4EFmutDREAM in HEK293 cells. A different effect of Ca²⁺ can be seen in the appearance of the 110K band of DREAM and 4EFmutDREAM. c, Delay-gel test showing the absence of effect of calcium on the DRE delay band obtained with the 4EFmutDREAM protein. Blocking of the calcium of the DRE delay band obtained with the native DREAM protein is shown for comparison. d, Western blot test of nuclear extract of DREAM in the absence or presence of calcium.

Fig. 4. DREAM is a Ca²⁺-dependent transcriptional repressor. a, Repression by DREAM on various reporters containing the DRE, pHD1CAT and TKDRECAT sequence, after transient cotransfection in HEK293 cells. Lack of repression on the mutated reporter pHD1mutDRECAT is also shown. Each bar in the diagram represents the relation between the CAT activities for each reporter cotransfected with DREAM or with empty expression vector pcDNA3. b, Repression by DREAM (black bars) or by 4EFmutDREAM (hatched bars) of basal transcription of pTKDRECAT after transient cotransfection in HEK293 cells. The repressor activity of DREAM is blocked by stimulation with calcium, whereas caffeine (10 mM) does not block repression mediated by 4EFmutDREAM. Treatment with forskolin (10 µM) did not block repression by DREAM of the reporter TKDRECAT, whereas with thapsigargin (1 µM) derepression by caffeine was blocked completely. The bars represent the relative CAT activity compared with that obtained after cotransfection with pcDNA3 (white bar) for each experimental condition: basal, caffeine, forskolin or caffeine plus thapsigargin. c, DREAM represses basal transcription of early induction genes. Transient transfection in HEK293 cells shows repression of the basal transcription of the reporters FC4CAT, c-junCAT and P2CAT. Mutation of the c-fosDRE site in the reporter FC4mDRECAT prevents repression of its basal transcription by DREAM. d, Transactivation of the reporter FC4CAT by caffeine in HEK293 cells is blocked by coexpression with the negative dominant mutant 4EFmutDREAM but not by DREAM. e, Alignment of the DRE site of the prodynorphin promoter and the DRE sites of the early gene promoters *c-fos, c-jun* and *ICER.* The consensus DRE sequence is shown at the bottom.

Fig. 5. Effect of αCREM in DRE-dependent transcription. a. Transactivation of the reporter pHD3CAT that contains a DRE site for αCREM after transient transfection in NB69 cells. The effect of treatment with forskolin is shown (black column), as well as the absence of effect of other isomorphs of CREM, ICER-I or CREB. b. Repression by DREAM (hatched column) on the reporter pHD3CAT in HEK293 cells is eliminated after cotransfection with αCREM. The absence of effect with the forms αCREMΔLZ, ICER-I or CREB is shown for comparison. The white columns represent the corresponding control transfection in absence of DREAM. c. The mutated forms of DREAM (DREAM L47-52V and DREAM L155V) do not interact with αCREM and their "unmodulable" repressor capacity remains unaffected. The black columns represent cotransfections with αCREM.

Fig. 6. DREAM interacts with CRE-binding proteins. a. Delay-gel test with a DRE probe shows the interaction between the proteins DREAM and αCREM produced in *E. coli* and their modulation by PKA-dependent phosphorylation. Absence of binding of the CRE binding proteins to the DRE probe is shown. Migration of the DRE-specific delay band is indicated with an arrowhead. b. Delay-gel test with a CRE probe shows the interaction between the CRE-binding proteins and with DREAM in increasing quantities (0, 0.2 and 1 µ g). Absence of binding of DREAM to the CRE probe in the absence of any CRE-binding protein is shown on the right-hand side of the diagram. The black and white arrows indicate the migration of the delayed bands of CRE obtained with different CRE-binding proteins in the absence of DREAM. The asterisks indicate the delay of the CRE bands in the presence of DREAM. The arrowheads show the migration of the delayed CRE bands obtained with ICER-I, which are not altered by DREAM. c. Effect of calcium (10 µM) and of PKA-dependent phosphorylation on the interaction between the proteins DREAM (2 µg) or CREB or αCREM (1 µg) using a CRE probe. The asterisks indicate the maximum delay obtained with DREAM in the absence of calcium and PKA.

Fig. 7. CRE-dependent transcription regulated by DREAM is Ca²⁺-dependent. a. Repression, by EFmutDREAM, of the transactivation of pSomCAT induced by caffeine after transient cotransfection of HEK293 cells. The values express the ratio obtained from comparing the levels obtained from induction of CAT activity in untreated cultures transfected with pCDNA3 empty expression vectors. The white columns represent the control values and the black columns represent the values after stimulation with caffeine. b. Rapid induction of the form fosfoCREB in HEK293 cells after caffeine. The levels of unphosphorylated CREB are not altered by caffeine. c. DREAM-dependent calcium blocking of the recruitment of CBP by CREB. Transactivation of the reporter pG5CAT by pGAL4-CREBΔLZ and the CBP coactivator after Ca²⁺ stimulation (black columns) is blocked in the presence of EFmutDREAM. d. The inability of EFmutDREAM to block the CBP-dependent transactivation of the pG5CAT reporter by pGAL4-cJunΔLZ after Ca²⁺ stimulation (black columns). In each group, the values are standardized with respect to cultures cotransfected with pCDNA3 in the absence of transfection with CBP and without caffeine treatment.

### Examples

### 1. Transfection of DREAM represses the basal transcription of promoters containing the DRE sequence in the absence of calcium.

To analyse the effect of DREAM on the transcription of genes containing a DRE site in its promoter, we cotransfected an expression vector of DREAM together with reporter plasmids with and without DRE elements. The tests were carried out in HEK293 cells lacking DREAM expression and transfection was effected using precipitates of calcium phosphate. Twelve hours after transfection, the precipitates of calcium phosphate were withdrawn, replacing them with fresh culture medium (DMEM-Glutamaxl-GIBCO-, supplemented with 10% of foetal calf serum and antibiotics). Twenty-four hours after transfection, the cells were collected and CAT activity was tested by standard procedures (Gorman, C.M., L.F. Moffat, and B.H. Howard. 1982. Recombinant genomes which express chloramphenicol acetyltransferase in mammalian cells. *Mol. Cell. Biol.* **2:**1044-1051). In those cases in which treatment with caffeine was carried out to release calcium ions from the intracellular deposits, the treatment was performed six hours before collecting the cells. The tests were repeated 3 to 5 times, each of them in triplicate.

Overexpression of DREAM caused a significant decrease of the reporters pHD1CAT, pTKDRECAT, pJ1CAT, p2CAT and FC4CAT, all of which contain a functional DRE sequence, whereas overexpression of DREAM did not affect basal transcription of the corresponding control reporters without DRE, pBLCAT3 and pBLCAT2, nor altered basal expression of the reporters pHD1mDRECAT and pFC4mDRECAT, derived from pHD1CAT and pFC4CAT, respectively, by specific mutation of the respective DRE sites (Fig. 4 a and c).

The repressor effect of DREAM on the transcription of pTKDRECAT and pFC4CAT was no longer observed when the test was analysed after administration of caffeine and subsequent increase in intracellular calcium levels (Fig. 4 b and d). However, derepression through increase of intracellular calcium was not observed when cotransfection was effected using negative dominant mutants of DREAM, which preserve their ability to bind to DRE but are not able to respond to the increase in calcium.

LIST OF SEQUENCES

<110> CONSEJO SUPERIOR DE INVESTIGACIONES CIENTIFICAS

<120> THE POLYPEPTIDE DREAM, USED AS Ca2+IONS-DEPENDENT

TRANSCRIPTIONAL REGULATOR AND ITS GENE CODING THEREFOR

<130>

<140>

<141>

<150> 9802667

<151> 1998-12-22

<160> 13

<170> PatentIn Ver. 2.1

<210> 1

<211> 46

<212> DNA

<213> Artificial sequence

<220>

<223> Double human sequence DynDRE

<400> SEQ ID NO. 1

caagccggag tcaaggaggc ccctgccgga gtcaaggagg cccctg 46

<210> 2

<211> 2890

<212> ADNc

<213> Homo sapiens

<220>

<221> CDS

<222> (103)..(870)

<400> SEQ ID NO. 2
gaattccggc aaacatgagg cagctgccag ccggcctggg cagtcttgtc tgcctcggct  60
gtgaagtggg gaggctggca acagttttct tcagcgccca gg acg cag ccg gct  114
Thr Gln Pro Ala
1
aag gaa gtg aca aag gcg tcg gac ggc agc ctc ctg ggg gac ctc ggg  162
Lys Glu Val Thr Lys Ala Ser Asp Gly Ser Leu Leu Gly Asp Leu Gly
5                   10                  15                  20
cac aca cca ctt agc aag aag gag ggt atc aag tgg cag agg ccg agg  210
His Thr Pro Leu Ser Lys Lys Glu Gly Ile Lys Trp Gln Arg Pro Arg
                25                  30                  35
ctc agc cgc cag gct ttg atg aga tgc tgc ctg gtc aag tgg atc ctg  258
Leu Ser Arg Gln Ala Leu Met Arg Cys Cys Leu Val Lys Trp Ile Leu
            40                  45                  50
tcc agc aca gcc cca cag ggc tca gat agc agc gac agt gag ctg gag  306
Ser Ser Thr Ala Pro Gln Gly Ser Asp Ser Ser Asp Ser Glu Leu Glu
        55                  60                  65
ctg tcc acg gtg cgc cac cag cca gag ggg ctg gac cag ctg cag gcc  354
Leu Ser Thr Val Arg His Gln Pro Glu Gly Leu Asp Gln Leu Gln Ala
    70                  75                  80
cag acc aag ttc acc aag aag gag ctg cag tct ctc tac agg ggc ttt  402
Gln Thr Lys Phe Thr Lys Lys Glu Leu Gln Ser Leu Tyr Arg Gly Phe
85                  90                  95                  100
aag aat gag tgt ccc acg ggc ctg gtg gac gaa gac acc ttc aaa ctc  450
Lys Asn Glu Cys Pro Thr Gly Leu Val Asp Glu Asp Thr Phe Lys Leu
                105                 110                 115
att tac gcg cag ttc ttc cct cag gga gat gcc acc acc tat gca cac  498
Ile Tyr Ala Gln Phe Phe Pro Gln Gly Asp Ala Thr Thr Tyr Ala His
            120                 125                 130
ttc ctc ttc aac gcc ttt gat gcg gac ggg aac ggg gcc atc cac ttt  546
Phe Leu Phe Asn Ala Phe Asp Ala Asp Gly Asn Gly Ala Ile His Phe
        135                 140                 145
gag gac ttt gtg gtt ggc ctc tcc atc ctg ctg cgg ggc aca gtc cac  594
Glu Asp Phe Val Val Gly Leu Ser Ile Leu Leu Arg Gly Thr Val His
    150                 155                 160
gag aag ctc aag tgg gcc ttt aat ctc tac gac att aac aag gat ggc  642
Glu Lys Leu Lys Trp Ala Phe Asn Leu Tyr Asp Ile Asn Lys Asp Gly
165                 170                 175                 180
tac gtc acc aaa gag gag atg ctg gcc atc atg aag tcc atc tat gac  690
Tyr Val Thr Lys Glu Glu Met Leu Ala Ile Met Lys Ser Ile Tyr Asp
                185                 190                 195
atg atg ggc cgc cac acc tac ccc atc ctg cag gag gac gcg ccg gcg  738
Met Met Gly Arg His Thr Tyr Pro Ile Leu Gln Glu Asp Ala Pro Ala
            200                 205                 210
gag cac gtg gag agg ttc ttc gag aaa atg gac cgg aac cag gat ggg  786
Glu His Val Glu Arg Phe Phe Glu Lys Met Asp Arg Asn Gln Asp Gly
        215                 220                 225
gta gtg acc att gaa gag ttc ctg gag gcc tgt cag aag gat gag aac  834
Val Val Thr Ile Glu Glu Phe Leu Glu Ala Cys Gln Lys Asp Glu Asn
    230                 235                 240
atc atg agc tcc atg cag ctg ttt gag aat gtc atc taggacacgt  880
Ile Met Ser Ser Met Gln Leu Phe Glu Asn Val Ile
245                 250                 255
ccaaaggagt gcatggccac agccacctcc acccccaaga aacctccatc ctgccaggag 940
cagcctccaa gaaactttta aaaaatagat ttgcaaaaag tgaacagatt gctacacaca 1000
cacacacaca cacacacaca cacacacaca gccattcatc tgggctggca gaggggacag 1060
agttcaggga ggggctgagt ctggctaggg gccgagtcca gaggccccag ccagcccttc 1120

ccaggccagc gaggcgaggc tgcctctggg tgagtggctg acagagcagg tctgcaggcc 1180
accagctgct ggatgtcacc aagaaggggc tcgagtgccc tgcaggaggg tccaatcctc 1240
cggtcccacc tcgtcccgtt catccattct gctttcttgc cacacagtgg ccggcccagg 1300
ctcccctggt ctcctccccg tagccactct ctgcccacta cctatgcttc tagaaagccc 1360
ctcacctcag gaccccagag gaccagctgg ggggcagggg ggagaggggg taatggaggc 1420
caagcctgca gctttctgga aattcttccc tgggggtccc agtatcccct gctactccac 1480
tgacctggaa gagctgggta ccaggccacc cactgtgggg caagcctgag tggtgagggg 1540
ccactggcat cattctccct ccatggcagg aaggcggggg atttcaagtt tagggattgg 1600
gtcgtggtgg agaatctgag ggcactctgc cagctccaca ggtggatgag cctctccttg 1660
ccccagtcct ggttcagtgg gaatgcagtg ggtggggctg tacacaccct ccagcacaga 1720
ctgttccctc caaggtcctc ttaggtcccg gggaggaacg tggttcagag actggcagcc 1780
agggagcccg gggcagagct cagaggagtc tgggaagggg cgtgtccctc ctcttcctgt 1840
agtgcccctc ccatggccca gcagcttggc tgagcccctc tcctgaagca gctgtgcgcc 1900
gtccctctgc cttgcacaaa aagcacaaga cattccttag cagctcagcg cagccctagt 1960
gggagcccag cacactgctt ctcggaggcc aggccctcct gctggctgag cttgggcccg 2020
gtggccccaa tatggtggcc ctggggaaga ggccttgggg gtctgctctg tgcctgggat 2080
cagtggggcc ccaaagccca gcccggctga ccaacattca aaagcacaaa ccctggggac 2140
tctgcttggc tgtcccctcc atctggggat ggagaatgca gcccaaagct ggagccaatg 2200
gtgagggctg agagggctgt ggctgggtgg tcagcagaaa ccccaggagg agagagatgc 2260
tgctcccgcc tgattggggc ctcacccaga aggaacccgg tcccagccgc atggcccctc 2320
caggaacatt cccacataat acattccatc acagccagcc cagctccact cagggctggc 2380
ccggggagtc cccgtgtgcc ccaagaggct agccccaggg tgagcagggc cctcagagga 2440
aaggcagtat ggcggaggcc atgggggccc ctcggcattc acacacagcc tggcctcccc 2500
tgcggagctg catggacgcc tggctccagg ctccaggctg actggggcct ctgcctccag 2560
gagggcatca gctttccctg gctcagggat cttctccctc ccctcacccg ctgcccagcc 2620
ctcccagctg atgtcactct gcctctaagc caaggcctca ggagagcatc accaccacac 2680
cctgcggcct tgccttgggg ccagactggc tgcacagccc aaccaggagg ggtctgcctc 2740
ccacgctggg acacagaccg gccgcatgtc tgcatggcag aagcgtctcc cttgccacgg 2800
cctgggaggg tggttcctgt tctcagcatc cactaatatt cagtcctgta tattttaata 2860
aaataaactt gacaaaggaa aaaaaaaccg 2890

<210> 3

<211> 256

<212> PRT

<213> Homo sapiens

<220>

<221> CA-BINB

<223>(102)..(113)

<220>

<221> CA-BINB

<223>(139)..(151)

<220>

<221> CA-BINB

<223>(175)..(186)

<220>

<221> CA-BINB

<223>(223)..(234)

<400> SEQ ID NO. 3

Thr Gln Pro Ala Lys Glu Val Thr Lys Ala Ser Asp Gly Ser Leu Leu
1               5                   10                  15
Gly Asp Leu Gly His Thr Pro Leu Ser Lys Lys Glu Gly Ile Lys Trp
            20                  25                  30
Gln Arg Pro Arg Leu Ser Arg Gln Ala Leu Met Arg Cys Cys Leu Val
        35                  40                  45
Lys Trp Ile Leu Ser Ser Thr Ala Pro Gln Gly Ser Asp Ser Ser Asp
    50                  55                  60
Ser Glu Leu Glu Leu Ser Thr Val Arg His Gln Pro Glu Gly Leu Asp
65                  70                  75                  80
Gln Leu Gln Ala Gln Thr Lys Phe Thr Lys Lys Glu Leu Gln Ser Leu
                85                  90                  95
Tyr Arg Gly Phe Lys Asn Glu Cys Pro Thr Gly Leu Val Asp Glu Asp
            100                 105                 110
Thr Phe Lys Leu Ile Tyr Ala Gln Phe Phe Pro Gln Gly Asp Ala Thr
        115                 120                 125
Thr Tyr Ala His Phe Leu Phe Asn Ala Phe Asp Ala Asp Gly Asn Gly
    130                 135                 140
Ala Ile His Phe Glu Asp Phe Val Val Gly Leu Ser Ile Leu Leu Arg
145                 150                 155                 160
Gly Thr Val His Glu Lys Leu Lys Trp Ala Phe Asn Leu Tyr Asp Ile
                165                 170                 175
Asn Lys Asp Gly Tyr Val Thr Lys Glu Glu Met Leu Ala Ile Met Lys
            180                 185                 190
Ser Ile Tyr Asp Met Met Gly Arg His Thr Tyr Pro Ile Leu Gln Glu
        195                 200                 205
Asp Ala Pro Ala Glu His Val Glu Arg Phe Phe Glu Lys Met Asp Arg
    210                 215                 220
Asn Gln Asp Gly Val Val Thr Ile Glu Glu Phe Leu Glu Ala Cys Gln
225                 230                 235                 240
Lys Asp Glu Asn Ile Met Ser Ser Met Gln Leu Phe Glu Asn Val Ile
                245                 250                 255

<210> 4

<211> 12

<212> PRT

<213> Homo sapiens

<400> SEQ ID NO. 4
Asn Glu Cys Pro Thr Gly Leu Val Asp Glu Asp Thr
1               5                   10

<210> 5
<211> 12
<212> PRT
<213> Homo sapiens

<400> SEQ ID NO. 5
Asp Ala Asp Gly Asn Gly Ala Ile His Phe Glu Asp
1               5                   10

<210> 6
<211> 12
<212> PRT
<213> Homo sapiens

<400> SEQ ID NO. 6
Asp Ile Asn Lys Asp Gly Tyr Val Thr Lys Glu Glu
1               5                   10

<210> 7
<211> 12
<212> PRT
<213> Homo sapiens

<400> SEQ ID NO. 7
Asp Arg Asn Gln Asp Gly Val Val Thr Ile Glu Glu
1               5                   10

<210> 8

<211> 12

<212> PRT

<213> Homo sapiens

<400> SEQ ID NO. 8

Ala Ala Ala Gly Asn Gly Ala Ile His Phe Glu Asp

1               5                   10

<210> 9

<211> 12

<212> PRT

<213> Homo sapiens

<400> SEQ ID NO. 9

Ala Ile Ala Lys Asp Gly Tyr Val Thr Lys Glu Glu

1               5                   10

<210> 10

<211> 12

<212> PRT

<213> Homo sapiens

<400> SEQ ID NO. 10

Ala Arg Ala Gln Asp Gly Val Val Thr Ile Glu Glu

1               5                   10

<210> 11

<211> 2886

<212> DNA

<213> Homo sapiens

<220>

<223> Antisense sequence of DREAM

<400> SEQ ID NO. 11

cggttttttt ttcctttgtc aagtttattt tattaaaata tacaggactg aatattagtg 60
gatgctgaga acaggaacca ccctcccagg ccgtggcaag ggagacgctt ctgccatgca 120
gacatgcggc cggtctgtgt cccagcgtgg gaggcagacc cctcctggtt gggctgtgca 180
gccagtctgg ccccaaggca aggccgcagg gtgtggtggt gatgctctcc tgaggccttg 240
gcttagaggc agagtgacat cagctgggag ggctgggcag cgggtgaggg gagggagaag 300
atccctgagc cagggaaagc tgatgccctc ctggaggcag aggccccagt cagcctggag 360
cctggagcca ggcgtccatg cagctccgca ggggaggcca ggctgtgtgt gaatgccgag 420
gggcccccat ggcctccgcc atactgcctt tcctctgagg gccctgctca ccctggggct 480
agcctcttgg ggcacacggg gactccccgg gccagccctg agtggagctg ggctggctgt 540
gatggaatgt attatgtggg aatgttcctg gaggggccat gcggctggga ccgggttcct 600
tctgggtgag gccccaatca ggcgggagca gcatctctct cctcctgggg tttctgctga 660
ccacccagcc acagccctct cagccctcac cattggctcc agctttgggc tgcattctcc 720
atccccagat ggaggggaca gccaagcaga gtccccaggg tttgtgcttt tgaatgttgg 780
tcagccgggc tgggctttgg ggccccactg atcccaggca cagagcagac ccccaaggcc 840
tcttccccag ggccaccata ttggggccac cgggcccaag ctcagccagc aggagggcct 900
ggcctccgag aagcagtgtg ctgggctccc actagggctg cgctgagctg ctaaggaatg 960
tcttgtgctt tttgtgcaag gcagagggac ggcgcacagc tgcttcagga gaggggctca 1020
gccaagctgc tgggccatgg gaggggcact acaggaagag gagggacacg ccccttccca 1080
gactcctctg agctctgccc cgggctccct ggctgccagt ctctgaacca cgttcctccc 1140
cgggacctaa gaggaccttg gagggaacag tctgtgctgg agggtgtgta cagccccacc 1200
cactgcattc ccactgaacc aggactgggg caaggagagg ctcatccacc tgtggagctg 1260
gcagagtgcc ctcagattct ccaccacgac ccaatcccta aacttgaaat cccccgcctt 1320
cctgccatgg agggagaatg atgccagtgg cccctcacca ctcaggcttg ccccacagtg 1380
ggtggcctgg tacccagctc ttccaggtca gtggagtagc aggggatact gggaccccca 1440
gggaagaatt tccagaaagc tgcaggcttg gcctccatta ccccctctcc cccctgcccc 1500
ccagctggtc ctctggggtc ctgaggtgag gggctttcta gaagcatagg tagtgggcag 1560
agagtggcta cggggaggag accaggggag cctgggccgg ccactgtgtg gcaagaaagc 1620
agaatggatg aacgggacga ggtgggaccg gaggattgga ccctcctgca gggcactcga 1680
gccccttctt ggtgacatcc agcagctggt ggcctgcaga cctgctctgt cagccactca 1740
cccagaggca gcctcgcctc gctggcctgg gaagggctgg ctggggcctc tggactcggc 1800
ccctagccag actcagcccc tccctgaact ctgtcccctc tgccagccca gatgaatggc 1860
tgtgtgtgtg tgtgtgtgtg tgtgtgtgtg tgtgtgtagc aatctgttca ctttttgcaa 1920
atctattttt taaaagtttc ttggaggctg ctcctggcag gatggaggtt tcttgggggt 1980
ggaggtggct gtggccatgc actcctttgg acgtgtccta gatgacattc tcaaacagct 2040
gcatggagct catgatgttc tcatccttct gacaggcctc caggaactct tcaatggtca 2100
ctaccccatc ctggttccgg tccattttct cgaagaacct ctccacgtgc tccgccggcg 2160
cgtcctcccg caggatgggg taggtgtggc ggcccatcat gtcatagatg gacttcatga 2220
tggccagcat ctcctctttg gtgacgtagc catccttgtt aatgtcgtag agattaaagg 2280
cccacttgag cttctcgtgg actgtgcccc gcagcaggat ggagaggcca accacaaagt 2340
cctcaaagtg gatggccccg ttcccgtccg catcaaaggc gttgaaggaa gtgtgcatag 2400
gtggtggcat ctcctgaggg aagaactgcg cgtaaatgag tttgaaggtg tcttcgtcca 2460
ccaggcccgt gggacactca ttcttaaagc ccctgtagag agactgcagc tccttcttgg 2520
tgaacttggt ctgggcctgc agctggtcca gcccctctgg ctgatggcgc accgtggaca 2580
gctccagctc actgtcgctg ctatctgagc cctgtggggc tgtgctggac aggatccact 2640
tgaccaggca gcatctcatc aaagcctggc ggctgagctc ggcctctgcc acttgatacc 2700
ctccttcttg ctcagtggtg tgtgcccgag gtcccccagg aggctgccgt ccgacgcctt 2760
tgtcacttcc ttgaccggct gcgtcctggg cgctgaagaa aactgttgcc agcctcccca 2820
cttcgcagcc gaggcagaca agactgccca ggccggctgg cagctgcctc atgtttgccg 2880
gaattc 2886

<210> 12

<211> 256

<212> PRT

<213> Artificial Sequence

<220>

<223> Mutated human DREAM protein, in which the original

amino acids Leucine-47 and -52 have been replaced by Valine

<400> SEQ ID NO. 12

Thr Gln Pro Ala Lys Glu Val Thr Lys Ala Ser Asp Gly Ser Leu Leu
1               5                   10                  15
Gly Asp Leu Gly His Thr Pro Leu Ser Lys Lys Glu Gly Ile Lys Trp
            20                  25                  30
Gln Arg Pro Arg Leu Ser Arg Gln Ala Leu Met Arg Cys Cys Val Val
        35                  40                  45
Lys Trp Ile Val Ser Ser Thr Ala Pro Gln Gly Ser Asp Ser Ser Asp
    50                  55                  60
Ser Glu Leu Glu Leu Ser Thr Val Arg His Gln Pro Glu Gly Leu Asp
65                  70                  75                  80
Gln Leu Gln Ala Gln Thr Lys Phe Thr Lys Lys Glu Leu Gln Ser Leu
                85                  90                  95
Tyr Arg Gly Phe Lys Asn Glu Cys Pro Thr Gly Leu Val Asp Glu Asp
            100                 105                 110
Thr Phe Lys Leu Ile Tyr Ala Gln Phe Phe Pro Gln Gly Asp Ala Thr
        115                 120                 125
Thr Tyr Ala His Phe Leu Phe Asn Ala Phe Asp Ala Asp Gly Asn Gly
    130                 135                 140
Ala Ile His Phe Glu Asp Phe Val Val Gly Leu Ser Ile Leu Leu Arg
145                 150                 155                 160
Gly Thr Val His Glu Lys Leu Lys Trp Ala Phe Asn Leu Tyr Asp Ile
                165                 170                 175
Asn Lys Asp Gly Tyr Val Thr Lys Glu Glu Met Leu Ala Ile Met Lys
            180                 185                 190
Ser Ile Tyr Asp Met Met Gly Arg His Thr Tyr Pro Ile Leu Gln Glu
        195                 200                 205
Asp Ala Pro Ala Glu His Val Glu Arg Phe Phe Glu Lys Met Asp Arg
    210                 215                 220
Asn Gln Asp Gly Val Val Thr Ile Glu Glu Phe Leu Glu Ala Cys Gln
225                 230                 235                 240
Lys Asp Glu Asn Ile Met Ser Ser Met Gln Leu Phe Glu Asn Val Ile
                245                 250                 255

<210> 13

<211> 256

<212> PRT
<213> Artificial Sequence
<220>
<223> Mutated human DREAM protein, in which the original
amino acid Leucine-155 has been replaced with Valine

<400> SEQ ID NO. 13

Thr Gln Pro Ala Lys Glu Val Thr Lys Ala Ser Asp Gly Ser Leu Leu
1               5                   10                  15
Gly Asp Leu Gly His Thr Pro Leu Ser Lys Lys Glu Gly Ile Lys Trp
            20                  25                  30
Gln Arg Pro Arg Leu Ser Arg Gln Ala Leu Met Arg Cys Cys Leu Val
        35                  40                  45
Lys Trp Ile Leu Ser Ser Thr Ala Pro Gln Gly Ser Asp Ser Ser Asp
    50                  55                  60
Ser Glu Leu Glu Leu Ser Thr Val Arg His Gln Pro Glu Gly Leu Asp
65                  70                  75                  80
Gln Leu Gln Ala Gln Thr Lys Phe Thr Lys Lys Glu Leu Gln Ser Leu
                85                  90                  95
Tyr Arg Gly Phe Lys Asn Glu Cys Pro Thr Gly Leu Val Asp Glu Asp
            100                 105                 110
Thr Phe Lys Leu Ile Tyr Ala Gln Phe Phe Pro Gln Gly Asp Ala Thr
        115                 120                 125
Thr Tyr Ala His Phe Leu Phe Asn Ala Phe Asp Ala Asp Gly Asn Gly
    130                 135                 140
Ala Ile His Phe Glu Asp Phe Val Val Gly Val Ser Ile Leu Leu Arg
145                 150                 155                 160
Gly Thr Val His Glu Lys Leu Lys Trp Ala Phe Asn Leu Tyr Asp Ile
                165                 170                 175
Asn Lys Asp Gly Tyr Val Thr Lys Glu Glu Met Leu Ala Ile Met Lys
            180                 185                 190
Ser Ile Tyr Asp Met Met Gly Arg His Thr Tyr Pro Ile Leu Gln Glu
        195                 200                 205
Asp Ala Pro Ala Glu His Val Glu Arg Phe Phe Glu Lys Met Asp Arg
    210                 215                 220
Asn Gln Asp Gly Val Val Thr Ile Glu Glu Phe Leu Glu Ala Cys Gln
225                 230                 235                 240
Lys Asp Glu Asn Ile Met Ser Ser Met Gln Leu Phe Glu Asn Val Ile
                245                 250                 255

## Claims

1. Gene and native polypeptide and mutated forms of DREAM, their use as specific transcription silencer at the level of DRE sequences, as target protein for the identification of new therapeutic compounds and regulator of CRE-dependent gene expression.

2. Polypeptide **characterized in that** it contains the sequence of 256 amino acids SEQ ID NO3.

3. Polypeptide according to Claim 2, **characterized in that** it comprises the sequence SEQ ID NO3, which extends from the end comprising the amino acid in position 1 to that comprising the end in position 256 and **in that** it exhibits a transcription repressor capacity that can be modulated.

4. Polypeptide **characterized in that** it has sufficient sequence homology with a polypeptide according to any one of Claims 1 to 3 and **in that** it binds to DRE.

5. Sequence of nucleotides **characterized in that** it contains or comprises a chain of nucleotides that codes for a polypeptide according to any one of Claims 2 to 4.

6. Sequence of nucleotides according to Claim 5, **characterized in that** it contains or comprises the chain of nucleotides of SEQ ID2, that extends from the end consisting of nucleotide 1 to that consisting of the end 2890.

7. Recombinant vector **characterized in that** it contains a sequence of nucleotides according to either of the Claims 5 and 6.

8. Recombinant vector according to Claim 7,
**characterized in that** it contains the elements necessary for promoting the expression of a polypeptide according to any one of Claims 1 to 4.

9. Transfected host cell **characterized in that** it contains a recombinant vector according to either of Claims 7 and 8.

10. Transfected host cell according to Claim 9, **characterized in that** the protein αCREM is also expressed in it.

11. Transfected host cell according to either of Claims 9 and 10, **characterized in that** expression of the polypeptide(s) is constitutive or induced by an active substance.

12. Transfected host cell according to Claim 11, **characterized in that** it is an embryonic cell.

13. Method that makes it possible to identify an active compound in the treatment of pathologic processes from its capacity for blocking or activating the DREAM polypeptide according to any one of Claims 1 to 4, **characterized in that** it comprises the following stages:
a) addition of the said compound to a transfected host cell according to any one of Claims 9 to 12,
b) determination of the change, either blocking or stimulation, in the repressor activity of gene expression of this polypeptide,
c) and selection of the said compound for the treatment of the said processes if the said transfected cells exhibit blocking or stimulation of the repressor activity of gene expression of this DREAM polypeptide.

14. Use of a transfected host cell according to Claim 12 for the development of transgenic animals, **characterized in that** expression of the polypeptide is constitutive or tissue-specific and cellular and **in that** they exhibit pathologic processes caused by alteration of gene expression of DREAM.

15. Method that makes it possible to identify an active compound in the treatment of pathologic processes from its capacity for blocking or activating the DREAM polypeptide according to any one of Claims 1 to 4, **characterized in that** it comprises the following stages:
a) administration of the said compound to a transgenic animal according to Claim 14,
b) determination of the decrease or total elimination of the symptoms of the pathologic processes,
c) and selection of the said compound for the treatment of the said processes if there is a decrease or total elimination of the symptoms of the pathologic processes.

16. Use of the compounds selected according to either of Claims 13 and 15 in the treatment of human pathologic processes, **characterized in that** they belong to the following group: neurodegenerative diseases and tumour-forming, autoimmune and inflammatory processes and learning disorders.

17. DREAM polypeptide according to any one of Claims 1 to 4, **characterized in that** it is a mutated form thereof and **in that** it exhibits transcriptional repressor activity that cannot be modulated.

18. DREAM polypeptide according to Claim 17,
**characterized in that** the mutation consists of the substitution, in at least one of the four EF-hand domains of this polypeptide, of the first (X) and third (Y) amino acids of the EF-hand domains of this polypeptide responsible for binding to Ca²⁺ (SEQ ID N8, SEQ ID N9 and SEQ ID N10).

19. DREAM polypeptide according to Claim 17,
**characterized in that** the mutation consists of the substitution in at least one of the amino acids involved in the interaction with the αCREM protein and by one of the following amino acid sequences: SEQ ID NO12 and SEQ ID NO13.

20. DREAM polypeptide, **characterized in that** it contains any one of the possible combinations of mutations according to either of Claims 18 and 19.

21. Nucleotide sequence, **characterized in that** it contains or is comprised of a chain of nucleotides that codes for a polypeptide according to any one of Claims 17 to 20.

22. Recombinant expression vector, **characterized in that** it contains a sequence of nucleotides according to Claim 21.

23. Recombinant vector according to Claim 22, **characterized in that** it contains the elements necessary for promoting the expression of a polypeptide according to any one of Claims 17 to 20 in a host cell.

24. Transfected host cell, **characterized in that** it contains a recombinant vector according to either Claim 22 or 23.

25. Transfected host cell according to Claim 24, **characterized in that** expression of the polypeptide is constitutive or induced by an active substance.

26. Transfected host cell according to Claim 25, **characterized in that** it is an embryonic cell.

27. Use of a transfected host cell according to Claim 26 for the development of transgenic animals, **characterized in that** expression of the polypeptide is constitutive or tissue-specific or of the cellular type and **in that** they exhibit pathologic processes caused by alteration of the activity of DREAM.

28. Sequence of nucleotides, **characterized in that** it contains or is comprised of the chain of nucleotides SEQ ID 11 and **in that** it undergoes complementary binding to the messenger RNA of DREAM.

29. Recombinant expression vector, **characterized in that** it contains a sequence according to Claim 27.

30. Transfected host cell, **characterized in that** it contains a recombinant vector according to Claim 29 and **in that** the expression of DREAM is suppressed.

31. Use of the transfected host cell according to Claim 30 for the expression of DRE-regulated genes.

32. Use of the recombinant vectors according to either of Claims 22 and 23 in gene therapy for provoking selective gene silencing of cell populations liable to cell proliferation, cellular degeneration, autoimmune processes or processes of hyperstimulation.

33. Use of the polypeptides according to either of Claims 17 and 18 in the regulation of CRE-dependent gene expression.

34. Monoclonal or polyclonal antibodies, **characterized in that** they recognize a polypeptide according to any one of Claims 1 to 4.

35. Use of the antibodies according to Claim 34 for purifying this DREAM polypeptide, for diagnosis or for therapeutic purposes.
